# EUROPEAN PATENT APPLICATION

(11) **EP 4 477 151 A1**
(43) Date of publication of application: **18.12.2024**
(21) Application number: 22933742.3
(22) Date of filing: 19.04.2022
(51) Int. Cl.: A61B 6/00, A61B 6/03, G16H 50/20

(54) **ARTIFICIAL INTELLIGENCE-BASED METHOD AND DEVICE FOR DETERMINING DENSITY VALUE WITH RESPECT TO SPECIFIC TISSUE OF NECK AND HEAD**

(30) Priority: 24.03.2022 KR 20220036713
(71) Applicant: 3D ONS, INC., Seoul 06023 (KR)
(72) Inventor: HONG, Nae Young, Seoul 06023 (KR); PANG, Jin, Seoul 06023 (KR)
(74) Representative: Walaski, Jan Filip
(86) International application number: PCT/KR2022/005623
(87) International publication number: WO 2023/182569

(57) **Abstract**

The present invention relates to a method for determining density values for specific tissues of the head and neck based on artificial intelligence. The method of the present invention includes an operation in which a computerized tomography (CT) image obtainer obtains a CT image of the head and neck of a patient from CT apparatuses of a plurality of different manufacturers, an operation in which a voxel image extractor extracts a plurality of voxel images for each density value section from the obtained CT image and merges the extracted plurality of voxel images, and an operation in which an image-of interest classifier classifies an image of interest by inputting the merged voxel image into an image-of-interest selection model and estimating a position value.

## Description

### [Technical Field]

The present invention relates to a method and apparatus for determining density values for specific tissues of the head and neck based on artificial intelligence, and more particularly, to a method and apparatus for determining density values for specific tissues of the head and neck based on artificial intelligence capable of determining density values for specific tissues of the head and neck by preprocessing a three-dimensional computerized tomography (CT) image for each density value section to extract a plurality of voxel images and inputting the extracted voxel images into an artificial intelligence model to classify an image of interest and then backtrack an order of the voxel images based on the image of interest.

### [Background Art]

Digital Imaging and Communications in Medicine (DICOM) data that is output from an apparatus should be input into a system to analyze data output from cone beam CT or general medical CT for patient diagnosis. Knowing density values for specific tissues of the head and neck makes it easy to identify information necessary for analysis when analyzing input data, but since density distribution is different for each manufacturer of cone beam CT or general medical CT, there is a problem in that analysis is difficult.

Korean Patent Registration No. 10-2033743 (Apparatus and method for CT image denoising based on deep learning) is one prior art document, but the prior art document only discloses a technology for performing CT image denoising using a deep learning model.

### [Disclosure]

### [Technical Problem]

The present invention is directed to providing a method and apparatus for determining density values for specific tissues of the head and neck based on artificial intelligence capable of determining density values for specific tissues of the head and neck based on artificial intelligence.

### [Technical Solution]

The present invention provides a method for determining density values for specific tissues of the head and neck based on artificial intelligence, the method including an operation in which a computerized tomography (CT) image obtainer obtains a CT image of the head and neck of a patient from CT apparatuses of a plurality of different manufacturers, an operation in which a voxel image extractor extracts a plurality of voxel images for each density value section from the obtained CT image and merges the extracted plurality of voxel images, and an operation in which an image-of interest classifier classifies an image of interest by inputting the merged voxel image into an image-of interest selection model and estimating a position value.

The present invention also provides an apparatus for determining density values for specific tissues of the head and neck based on artificial intelligence, the apparatus including a computerized tomography (CT) image obtainer configured to obtain a CT image of the head and neck of a patient from CT apparatuses of a plurality of different manufacturers, a voxel image extractor configured to extract a plurality of voxel images for each density value section from the obtained CT image and merge the extracted plurality of voxel images, an image-of-interest classifier configured to input the merged voxel image into an image-of interest selection model and estimate a position value, and a density value determiner configured to determine a density value by backtracking an order of the plurality of voxel images using the estimated position value.

### [Advantageous Effects]

According to the present invention, it is possible to determine density values for specific tissues of the head and neck from CT images output from various CT apparatuses regardless of manufacturer.

Also, since it is possible to classify a CT image by automatically dividing the CT image for each density value section, patient diagnosis and analysis can be conveniently provided.

### [Description of Drawings]

FIG. 1 is a flowchart for describing a method for determining density values for specific tissues of the head and neck based on artificial intelligence according to an embodiment of the present invention.
FIG. 2 is a block diagram for describing an apparatus for determining density values for specific tissues of the head and neck based on artificial intelligence according to an embodiment of the present invention.
FIGS. 3 and 4 are views for describing a method for extracting voxel images for each density value section according to an embodiment of the present invention.
FIG. 5 is a view for describing a method for determining a density value by classifying an image of interest according to an embodiment of the present invention.
FIG. 6 is a conceptual diagram for describing an image-of-interest selection model according to an embodiment of the present invention.

### [Modes of the Invention]

The specific structural or functional description is merely illustrative for the purpose of describing embodiments according to the concept of the present invention with respect to embodiments according to the concept of the present invention disclosed herein. Embodiments according to the concept of the present invention may be implemented in various forms, and may not be construed as limited to the embodiments set forth herein.

Since embodiments according to the concept of the present invention may be changed in various ways and have various forms, the embodiments are illustrated in the drawings and will be described in detail herein. However, it is not intended to limit the embodiments according to the concept of the present invention to specific disclosed forms, and the present invention includes all changes, equivalents, or substitutes included in the spirit and technical scope of the present invention.

Terms used herein are only used to describe specific embodiments and are not intended to limit the present invention. A singular expression includes a plural expression unless the context clearly indicates otherwise. In the present specification, terms such as "include" or "have" should be understood as indicating the presence of features, numbers, steps, operations, elements, parts, or combinations thereof and not excluding the possibility of the presence or addition of one or more other features, numbers, steps, operations, elements, parts, or combinations thereof in advance.

Hereinafter, embodiments of the present invention will be described in detail with reference to the accompanying drawings.

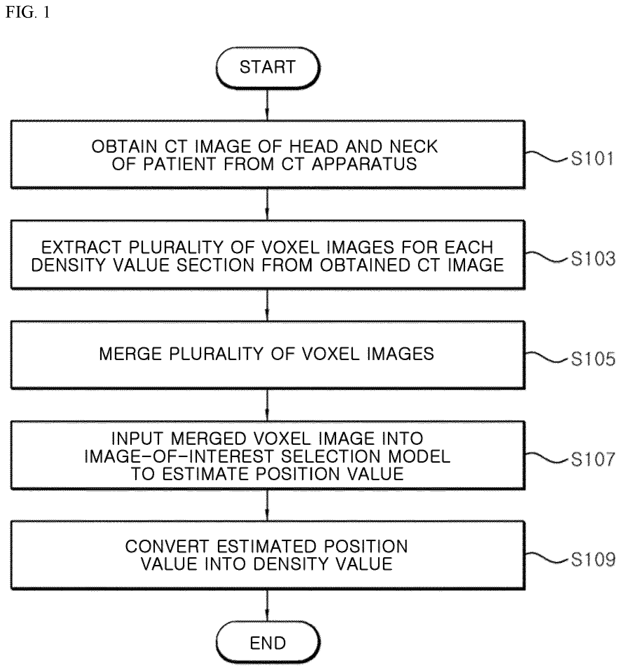

FIG. 1 is a flowchart for describing a method for determining density values for specific tissues of the head and neck based on artificial intelligence according to an embodiment of the present invention.

Referring to FIG. 1, a computerized tomography (CT) image obtainer of a density value determination device obtains a CT image of the head and neck of a patient from CT apparatuses of a plurality of different manufacturers (S101). A voxel image extractor of the density value determination device extracts a plurality of voxel images for each density value section from the obtained CT image (S103). Since a bone density value in the obtained CT image is unknown, a minimum value and a maximum value of a density value of CT image data may be designated to set a density value section. Here, a part where a numerical value of density suddenly increases when density values in CT image data are sequentially tested from the smallest density value may be designated as the minimum value, and a section where a numerical value of density exceeds a certain number when density values in CT image data are tested in the reverse order from the largest density value may be designated as the maximum value.

The voxel image extractor of the density value determination device merges the extracted plurality of voxel images (S105). An image-of interest classifier of the density value determination device inputs the merged voxel image into an image-of interest selection model and estimates a position value (S107). The image-of-interest classifier may estimate a position value of a desired two-dimensional image among two-dimensional images which are components of an extracted three-dimensional image. The image-of interest classifier may estimate a specific numerical value (for example, a depth value) from a three-dimensional image through a 3D convolutional neural network (CNN) regression model.

A density value determiner of the density value determination device determines a density value by backtracking an order of the plurality of voxel images based on the image of interest classified using the estimated position value (S107). The estimated specific numerical value may be converted into a specific density value through a process of backtracking using a value corresponding to a depth value. The density value determiner may derive a position value through the image-of-interest selection model and perform backtracking based on the position value to identify a density value. The backtracking involves performing calculation by mapping the position value derived by the image-of-interest selection model onto the maximum (max) value and the minimum (min) value of density. That is, the backtracking calculation formula is ((max-min)×(inferred value))+min. According to the present invention, when a model is allowed to learn to identify the density of a desired tissue such as teeth or skin in addition to bone, the density can be identified for a specific body tissue.

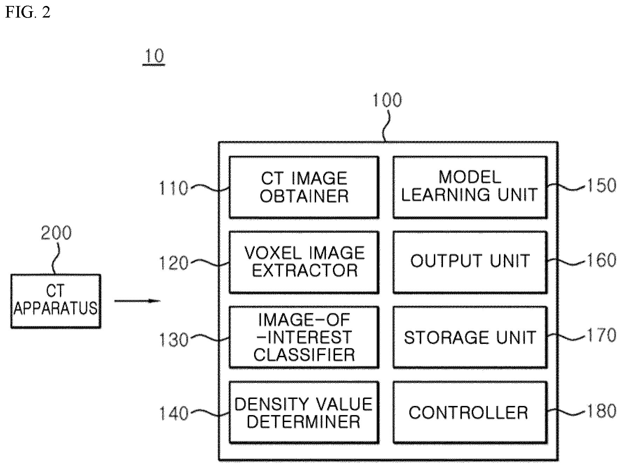

FIG. 2 is a block diagram for describing an apparatus for determining density values for specific tissues of the head and neck based on artificial intelligence according to an embodiment of the present invention.

Referring to FIG. 2, a density value determination device 100 includes a CT image obtainer 110, a voxel image extractor 120, an image-of interest classifier 130, a density value determiner 140, a model learning unit 150, an output unit 160, a storage unit 170, and a controller 180.

The CT image obtainer 110 obtains a CT image of the head and neck of a patient from a CT apparatus 200. In the present invention, although the CT image is described as a CT image of the head and neck of a patient, an imaging area of the patient is not limited to the head and neck. The CT image obtainer 110 may obtain a CT image of the head and neck of a patient from CT apparatuses of different manufacturers.

The voxel image extractor 120 may extract a plurality of voxel images for each density value section from the obtained CT image. The voxel image extractor 120 may preset density value sections and may perform preprocessing to extract voxel images for each set section. Here, a part where a numerical value of density suddenly increases when density values in CT image data are sequentially tested from the smallest density value may be designated as the minimum value of the density value sections, and a section where a numerical value of density exceeds a certain number when density values in CT image data are tested in the reverse order from the largest density value may be designated as the maximum value.

The number of voxel images extracted by the voxel image extractor 120 is not limited. The voxel image extractor 120 may sequentially assign numbers to the extracted voxel images from a voxel image with a low density to a voxel image with a high density. The extracted voxel images may be generated with a quality that allows the voxel images to be checked even when viewed by the human eye.

The voxel image extractor 120 may merge the extracted voxel images into a single three-dimensional voxel image.

The image-of interest classifier 130 may input the three-dimensional voxel image into an image-of-interest selection model and estimate a position value. The image-of-interest selection model is a CNN model that has learned learning data in advance in the model learning unit and is a model that can select an image of interest that is desired to be classified among a plurality of images. The image of interest is a voxel image having a density value of most interest among a plurality of extracted voxel images.

The image-of-interest selection model is designed to extract necessary features from an image received as an input as the image passes through a plurality of layers consisting of a convolution layer and a maxpooling layer and extract a feature value between 0 and 1 by a fully connected layer receiving the extracted features. A feature extraction module consisting of the convolution layer and the maxpooling layer is trained to perform a process of extracting optimized features to assist the fully connected layer, which is a numerical value extraction module, in determining a suitable numerical value. The fully connected layer of the numerical value extraction module that receives the optimized features as an input is trained to finally determine which numerical value among values between 0 and 1 to extract. The image-of-interest selection model may extract an input image as an appropriate numerical value through connection between the feature extraction module and the numerical value extraction module.

The density value determiner 140 may determine a density value by backtracking an order of the plurality of voxel images based on the image of interest classified using the estimated position value. The density value determiner 140 may determine a density value using a number assigned to a voxel image. The density value determiner may derive a position value through the image-of-interest selection model and perform backtracking based on the position value to identify a density value. The backtracking involves performing calculation by mapping the position value derived by the image-of-interest selection model onto the maximum (max) value and the minimum (min) value of density.

The model learning unit 150 learns the relations between the voxel images and the image of interest using the CNN-based image-of interest selection model.

The output unit 160 may output the density value determined by the density value determiner so that the density value is provided to the user.

The storage unit 170 may store the CT images obtained from the plurality of CT apparatuses, the extracted voxel images, the classified image of interest, and the determined density value.

The controller 180 may have operating software for controlling each component of the density value determination device installed therein to control the CT image obtainer, the voxel image extractor, the image-of-interest classifier, the density value determiner, the model learning unit, the output unit, and the storage unit.

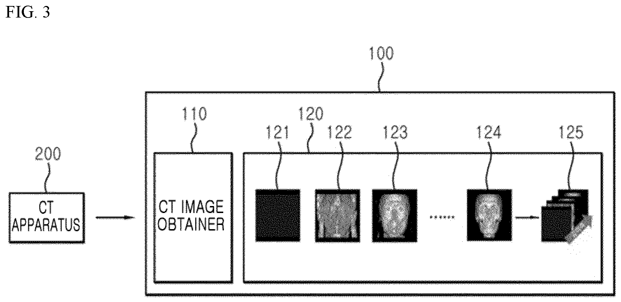

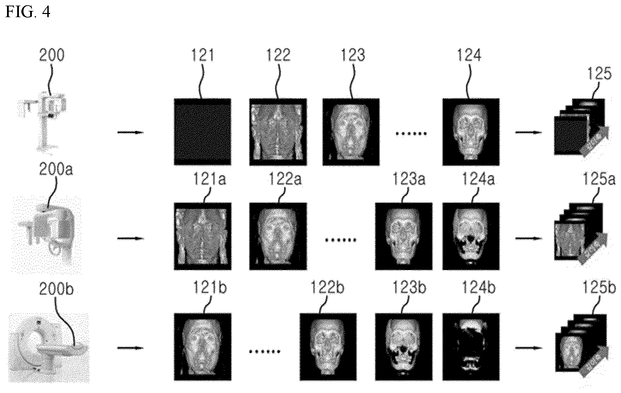

FIGS. 3 and 4 are views for describing a method for extracting voxel images for each density value section according to an embodiment of the present invention.

Referring to FIG. 3, the CT image obtainer 110 may obtain a CT image from the CT apparatus 200, and the voxel image extractor 120 may extract voxel images for each density value section. A first voxel image 121 may be extracted from a first section with a low density value, a second voxel image 122 may be extracted from a second section with a higher density value than the first section, a third voxel image 123 may be extracted from a third section with a higher density value than the second section, and a fourth voxel image 124 may be extracted from a fourth section with the highest density. The first to fourth voxel images are merged to generate a three-dimensional voxel image 125.

Referring to FIG. 4, CT images output from a plurality of CT apparatuses 200, 200a, and 200b of different manufacturers may have different density distributions, and the voxel image extractor 120 may extract voxel images for each preset density value section from the CT images having different density distributions.

As CT images obtained from a first CT apparatus 200, the first voxel image 121 may be extracted from the first section with a low density value, the second voxel image 122 may be extracted from the second section with a higher density value than the first section, the third voxel image 123 may be extracted from the third section with a higher density value than the second section, and the fourth voxel image 124 may be extracted from the fourth section with the highest density. The extracted plurality of voxel images are merged to generate the three-dimensional voxel image 125.

As CT images obtained from a second CT apparatus 200a, a first voxel image 121a may be extracted from a first section with a low density value, a second voxel image 122a may be extracted from a second section with a higher density value than the first section, a third voxel image 123a may be extracted from a third section with a higher density value than the second section, and a fourth voxel image 124a may be extracted from a fourth section with the highest density. The extracted plurality of voxel images are merged to generate a three-dimensional voxel image 125a. As CT images obtained from a third CT apparatus 200b, a first voxel image 121b may be extracted from a first section with a low density value, a second voxel image 122b may be extracted from a second section with a higher density value than the first section, a third voxel image 123b may be extracted from a third section with a higher density value than the second section, and a fourth voxel image 124b may be extracted from a fourth section with the highest density. The extracted plurality of voxel images are merged to generate a three-dimensional voxel image 125b.

Here, an image of interest that the user wants to select from the first CT apparatus 200 may be the fourth voxel image 124, an image of interest that the user wants to select from the second CT apparatus 200a may be the third voxel image 123a, and an image of interest that the user wants to select from the third CT apparatus 200b may be the second voxel image 122b. That is, even when the density distributions are different due to the manufacturers being different, voxel images may be extracted for each density value section by the voxel image extractor.

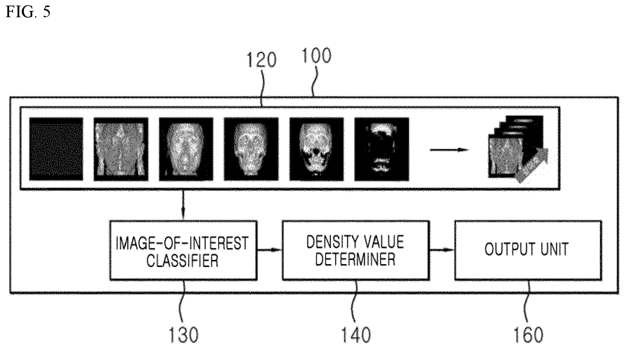

FIG. 5 is a view for describing a method for determining a density value by classifying an image of interest according to an embodiment of the present invention.

Referring to FIG. 5, the voxel image extractor 120 of the density value determination device 100 sequentially extracts voxel images from a voxel image with a low density value to a voxel image with a high density value, and the extracted plurality of voxel images are merged to generate a three-dimensional voxel image. The three-dimensional voxel image may be input into the image-of-interest selection model of the image-of-interest classifier to estimate a position value, and the density value determiner may perform backtracking based on the image of interest classified through the estimated position value to determine a density value. The determined density value may be output by the output unit and provided to the user.

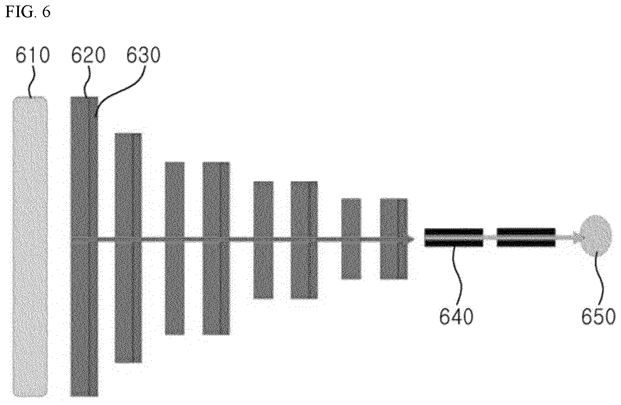

FIG. 6 is a conceptual diagram for describing an image-of-interest selection model according to an embodiment of the present invention. Referring to FIG. 6, when a merged three-dimensional voxel image 610 is input into the image-of interest selection model, the image-of interest selection model may extract necessary features therefrom as the image passes through a plurality of layers consisting of a convolution layer 620 and a maxpooling layer 630 and extract a feature value 650 between 0 and 1 by a fully connected layer 640 receiving the extracted features.

A feature extraction module consisting of the convolution layer 620 and the maxpooling layer 630 is trained to perform a process of extracting optimized features to assist the fully connected layer 640, which is a numerical value extraction module, in determining a suitable numerical value. The fully connected layer 640 of the numerical value extraction module that receives the optimized features as an input is trained to finally determine which numerical value among values between 0 and 1 to extract. The image-of-interest selection model may extract an input image as an appropriate numerical value through connection between the feature extraction module and the numerical value extraction module.

Although the present invention has been described above with reference to the embodiments illustrated in the drawings of the invention, the description is merely illustrative, and those of ordinary skill in the art should understand that various modifications and other equivalent embodiments are possible therefrom. Therefore, the true technical protection scope of the present invention should be defined by the technical spirit of the appended claims.

### [Description of reference numerals]

| | | | |
|---|---|---|---|
| 100: | density value determination device | 110: | CT image obtainer |
| 120: | voxel image extractor | 130: | image-of interest classifier |
| 140: | density value determiner | 150: | model learning unit |
| 160: | output unit | 170: | storage unit |
| 180: | controller | | |

## Claims

1. A method for determining density values for specific tissues of the head and neck based on artificial intelligence, the method comprising:
an operation in which a computerized tomography (CT) image obtainer obtains a CT image of the head and neck of a patient from CT apparatuses of a plurality of different manufacturers;
an operation in which a voxel image extractor extracts a plurality of voxel images for each density value section from the obtained CT image and merges the extracted plurality of voxel images; and
an operation in which an image-of-interest classifier classifies an image of interest by inputting the merged voxel image into an image-of-interest selection model and estimating a position value.

2. The method of claim 1, further comprising an operation in which a density value determiner determines a density value by backtracking an order of the plurality of voxel images using the estimated position value.

3. The method of claim 2, wherein the plurality of voxel images are sequentially output from a low density to a high density.

4. The method of claim 2, wherein the backtracking includes performing calculation by mapping the position value derived by the image-of-interest selection model onto the maximum (max) value and the minimum (min) value of density.

5. An apparatus for determining density values for specific tissues of the head and neck based on artificial intelligence, the apparatus comprising:
a computerized tomography (CT) image obtainer configured to obtain a CT image of the head and neck of a patient from CT apparatuses of a plurality of different manufacturers;
a voxel image extractor configured to extract a plurality of voxel images for each density value section from the obtained CT image and merge the extracted plurality of voxel images;
an image-of-interest classifier configured to input the merged voxel image into an image-of-interest selection model and estimate a position value; and
a density value determiner configured to determine a density value by backtracking an order of the plurality of voxel images using the estimated position value.
